# EUROPEAN PATENT APPLICATION

(11) **EP 4 342 477 A1**
(43) Date of publication of application: **27.03.2024**
(21) Application number: 22804494.7
(22) Date of filing: 20.04.2022
(51) Int. Cl.: A61K 31/716, A23L 33/145, A61K 31/736, A61K 36/06, A61K 36/064, A61P 37/04, A61P 43/00, C08B 37/00, C12N 1/16, C12N 1/18, C12P 19/04

(54) **IMMUNOSTIMULATOR AND GLUCAN COMPOSITION PRODUCTION METHOD**

(30) Priority: 21.05.2021 JP 2021086394
(71) Applicant: Asahi Group Holdings, Ltd., Tokyo 130-8602 (JP)
(72) Inventor: SHIMADA, Yu, Moriya-shi, Ibaraki 302-0106 (JP); YOSHIDA, Jumpei, Moriya-shi, Ibaraki 302-0106 (JP); MINAMI, Taichi, Moriya-shi, Ibaraki 302-0106 (JP); ISHIDA, Tetsuya, Moriya-shi, Ibaraki 302-0106 (JP); SHIRAI, Takeshi, Moriya-shi, Ibaraki 302-0106 (JP); IMAI, Yu, Moriya-shi, Ibaraki 302-0106 (JP)
(74) Representative: Müller-Boré & Partner Patentanwälte PartG mbB
(86) International application number: PCT/JP2022/018278
(87) International publication number: WO 2022/244580

(57) **Abstract**

Provided is an immunostimulator that contains β-glucan derived from yeast cell walls and has a high immunostimulating effect. The immunostimulator contains β-glucan derived from yeast cell walls and the β-glucan content is from 25% by mass to 45% by mass.

## Description

### TECHNICAL FIELD

The present invention relates to an immunostimulator and a method of producing a glucan composition.

### BACKGROUND ART

There is a recent and growing concern about the worldwide spread of drug-resistant bacteria. World Health Organization (WHO) points out the abuse of antibiotics for domestic animals as one of the causes, and alternatives to antibiotics, in other words, solutions to infections are demanded by the livestock industry. Enhancement of the immune function inherent in living organisms is effective as a countermeasure against infectious diseases, for which β-glucan, a well-recognized material with immunostimulating effect, is expected.

Glucan materials derived from yeasts utilized as immunostimulators are generally thought to have higher immunostimulating effect when they have a higher degree of purification (see, for example, Jung-Nam Lee et.al, Biosci. Biotechnol. Biochem., 65(4),837-841, 2001.). JP 2010-533479 A, for example, suggests a method of preparing glucan preparations by combining enzymatic treatment and acid/alkali hydrolysis treatment, which glucan preparations have a high glucan content and are considered suitable for industrial use.

### SUMMARY OF THE INVENTION

### PROBLEMS TO BE SOLVED BY THE INVENTION

Materials containing yeast-derived β-glucans are required to have higher immunostimulating effect. An aspect of the present invention aims to provide an immunostimulator comprising β-glucan derived from yeast cell walls, and having high immunostimulating effect.

### MEANS FOR SOLVING THE PROBLEMS

A first aspect is an immunostimulator containing β-glucan derived from yeast cell walls, wherein a β-glucan content is from 25% by mass to 45% by mass. In one embodiment, the immunostimulator may further contain mannan, wherein a mannan content in the immunostimulator may be from 5% by mass to 20% by mass, and wherein a ratio of the mannan content to the β-glucan content may be 0.1 or more and less than 1. In one embodiment, the immunostimulator may further contain α-glucan, wherein an α-glucan content in the immunostimulator may be from 7% by mass to 12% by mass, and wherein a ratio of the α-glucan content to the β-glucan content may be from 0.15 to 0.55. In one embodiment, the α-glucan may contain glycogen, wherein a glycogen content in the immunostimulator may be from 4% by mass to 10% by mass, and wherein a ratio of the glycogen content to the β-glucan content may be from 0.16 to 0.5.

A second aspect is an interleukin 8 production promoter containing β-glucan derived from yeast cell walls, wherein a β-glucan content is from 25% by mass to 45% by mass. In one embodiment, the interleukin 8 production promoter may further contain mannan, wherein a mannan content in the interleukin 8 production promoter may be from 5% by mass to 20% by mass, and wherein a ratio of the mannan content to the β-glucan content may be 0.1 or more and less than 1. In one embodiment, the interleukin 8 production promoter may further contain α-glucan, wherein an α-glucan content in the interleukin 8 production promoter may be from 7% by mass to 12% by mass, and wherein a ratio of the α-glucan content to the P-glucan content may be from 0.15 to 0.55. In one embodiment, the α-glucan may contain glycogen, wherein a glycogen content in the interleukin 8 production promoter may be from 4% by mass to 10% by mass, and wherein a ratio of the glycogen content to the β-glucan content may be from 0.16 to 0.5.

A third aspect is a method of producing a glucan composition, the method including: washing yeast cell walls under alkaline conditions to obtain a first treated material; heating the first treated material under alkaline conditions to obtain a second treated material; and collecting a third treated material as a solid content from the second treated material, wherein in the third treated material, the β-glucan content is from 25% by mass to 45% by mass. In one embodiment, the heating under alkaline conditions may be performed at a pH from 8 to 14 and at a temperature from 70°C to 110°C. In one embodiment, the yeast cell walls may contain cell walls derived from a brewing yeast or a baker's yeast. In one embodiment, the washing under alkaline conditions may include: adjusting the pH of a wash mixture containing yeast cell walls to a pH of 8 to 14, and separating the solid and liquid using a centrifuge with addition of water. In one embodiment, the third treated material may be collected by subjecting the second treated material to solid-liquid separation by centrifugation. In one embodiment, the glucan composition to be produced may be an immunostimulator or an interleukin 8 production promoter.

### EFFECT OF THE INVENTION

According to an aspect of the present invention, an immunostimulator containing β-glucan derived from yeast cell walls, and having high immunostimulating effect may be provided.

### BRIEF DESCRIPTION OF THE DRAWINGS

FIG. 1 shows the interleukin 8 production promoting effects of the glucan compositions according to Example 1 and Comparative Example 1.
FIG. 2 shows the interleukin 8 production promoting effects of the glucan compositions according to Example 1 and Comparative Examples 2 and 3.

### DETAILED DESCRIPTION OF THE INVENTION

The term "step" as used herein not only includes an independent step, but also includes a step that is even not clearly distinguishable from other steps as long as the desired purpose of the step is achieved. Unless otherwise specified, the amounts of components in a composition, when a plurality of substances corresponding to the components are present in the composition, mean the total amount of the plurality of substances present in the composition. Moreover, for the upper and lower limits of a numerical range described herein, any values illustrated as numerical ranges can be selected and combined. Embodiments of the present invention will be described in detail below. However, the embodiments described below exemplify immunostimulators and methods of producing a glucan composition to embody the technical idea of the present invention, and the present invention is not limited to the immunostimulators and methods of producing a glucan composition shown below.

### Immunostimulator

An immunostimulator is a glucan composition containing β-glucan derived from yeast cell walls, wherein the β-glucan content in the immunostimulator is, for example, from 25% by mass to 45% by mass. The immunostimulator according to the present embodiment contains a predetermined amount of β-glucan derived from yeast cell walls, thereby having an excellent immunostimulating effect. This is not only expected to reduce the incidence and progression of infectious diseases, such as in livestock, humans, and farmed fish, but also may provide means of reducing the threat of the emergence of drug-resistant bacteria.

The immunostimulating effect of an immunostimulator may be evaluated, for example, based on its effect to promote the production of cytokines involved in immunity, such as interleukins. Thus, the immunostimulator may be an interleukin production promoter, preferably an interleukin 8 production promoter.

The β-glucans constituting the immunostimulator are polymers in which D-glucoses are linked via glycosidic linkages, and have a β-1,3-linked linear main chain backbone and β-1,6-linked branched side chains. For example, the β-glucans constituting the immunostimulator are preferably derived from yeast cell walls and obtained by hydrolyzing yeast cell walls, and may be contained in a glucan composition produced by a method of producing a β-glucan composition as described later.

Preferably, the β-glucan content in the immunostimulator may be 28% by mass or more, 30% by mass or more, 34% by mass or more, or 35% by mass or more. Preferably, the β-glucan content in the immunostimulator may be 42% by mass or less, 40% by mass or less, 38% by mass or less, or 35% by mass or less. β-glucan contents within the range described above tend to result in higher immunostimulating effect. It is noted that the β-glucan content herein described is dry matter weight. Similarly, the mannan, α-glucan, glycogen, and other contents described below are also dry matter weights.

In addition to β-glucan, the immunostimulator may further contain mannan. When the immunostimulator contains mannan, the mannan content in the immunostimulator may be, for example, from 5% by mass to 20% by mass. The mannan content in the immunostimulator may be preferably 7% by mass or more, 9% by mass or more, or 10% by mass or more, and may be preferably 18% by mass or less, 16% by mass or less, 14% by mass or less, or 12% by mass or less. Mannan contents within the range described above tend to result in higher immunostimulating effect.

The ratio of the mannan content to the β-glucan content in the immunostimulator may be, for example, 0.1 or more and less than 1 from the viewpoint of promoting effects on the production of interleukins. The ratio of the mannan content to the β-glucan content in the immunostimulator may be preferably 0.2 or more, 0.25 or more, 0.28 or more, or 0.3 or more, and may be preferably 0.8 or less, 0.6 or less, 0.4 or less, or 0.35 or less.

In addition to β-glucan, the immunostimulator may further contain α-glucan. When the immunostimulator contains α-glucan, the α-glucan content in the immunostimulator may be, for example, from 7% by mass to 12% by mass. The α-glucan content in the immunostimulator may be preferably 8% by mass or more, 9% by mass or more, or 9.5% by mass or more, and may be preferably 18% by mass or less, 11% by mass or less, 10.5% by mass or less, or 10% by mass or less. α-glucan contents within the range described above tend to result in higher immunostimulating effect.

The ratio of the α-glucan content to the β-glucan content in the immunostimulator may be, for example, from 0.15 to 0.55 from the viewpoint of promoting effects on the production of interleukins. The ratio of the α-glucan content to the β-glucan content in the immunostimulator may be preferably 0.18 or more, 0.2 or more, 0.24 or more, or 0.26 or more, and may be preferably 0.5 or less, 0.4 or less, 0.3 or less, or 0.28 or less.

The immunostimulator may comprise glycogen as a part of the α-glucan content. When the immunostimulator comprises glycogen, the glycogen content in the immunostimulator may be, for example, from 4% by mass to 10% by mass. The glycogen content in the immunostimulator may be preferably 5% by mass or more, 6% by mass or more, or 6.4% by mass or more, and may be preferably 9% by mass or less, 8% by mass or less, 7% by mass or less, or 6.8% by mass or less. Glycogen contents within the range described above tend to result in higher immunostimulating effect.

The ratio of the glycogen content to the β-glucan content in the immunostimulator may be, for example, from 0.16 to 0.3 from the viewpoint of promoting effects on the production of interleukins. The ratio of the glycogen content to the β-glucan content in the immunostimulator may be preferably 0.17 or more, 0.18 or more, or 0.182 or more, and may be preferably 0.25 or less, 0.22 or less, 0.2 or less, or 0.19 or less.

The total content of the β-glucan and α-glucan in the immunostimulator may be, for example, from 35% by mass to 52% by mass. The total content of the β-glucan and α-glucan in the immunostimulator may be preferably 38% by mass or more, 40% by mass or more, 42% by mass or more, or 44% by mass or more, and may be preferably 50% by mass or less, 48% by mass or less, or 46% by mass or less. The total content of the β-glucan and α-glucan being within the range described above tends to results in higher immunostimulating effect.

The ratio of the β-glucan content to the total content of the β-glucan and α-glucan in the immunostimulator (hereinafter also referred to as "total glucan content") may be, for example, from 0.65 to 0.85, from the viewpoint of promoting effects on the production of interleukins. The ratio of the β-glucan content to the total glucan content in the immunostimulator may be preferably 0.68 or more, 0.7 or more, 0.72 or more, 0.74 or more, 0.76 or more, or 0.78 or more, and may be preferably 0.82 or less, 0.8 or less, or 0.79 or less.

The ratio of the α-glucan content to the total glucan content in the immunostimulator may be, for example, from 0.14 to 0.35 from the viewpoint of promoting effects on the production of interleukins. The ratio of the α-glucan content to the total glucan content in the immunostimulator may be preferably 0.16 or more, 0.18 or more, or 0.2 or more, and may be preferably 0.3 or less, 0.28 or less, 0.26 or less, 0.24 or less, or 0.22 or less.

The ratio of the mannan content to the total glucan content in the immunostimulator may be, for example, from 0.1 to 0.6 from the viewpoint of promoting effects on the production of interleukins. The ratio of the mannan content to the total glucan content in the immunostimulator may be preferably 0.14 or more, 0.18 or more, 0.2 or more, or 0.22 or more, and may be preferably 0.5 or less, 0.4 or less, 0.3 or less, 0.28 or less, or 0.26 or less.

The ratio of the glycogen content to the total glucan content in the immunostimulator may be, for example, from 0.11 to 0.3 from the viewpoint of promoting effects on the production of interleukins. The ratio of the glycogen content to the total glucan content in the immunostimulator may be preferably 0.11 or more, 0.12 or more, 0.13 or more, or 0.14 or more, and may be preferably 0.2 or less, 0.18% or less, 0.16 or less, or 0.15 or less.

The immunostimulator can exhibit high immunostimulating effect in spite of its relatively low level of purification of the β-glucan. This may be considered because, for example, experience of only low-intensity extraction under weak alkaline conditions results in effective concentration of active ingredients without excessively destroying the conformation of yeast cell walls, allowing β-glucan to maintain a necessary and sufficient morphology for immune responses. Alternatively, this may be considered because the immunostimulator contains a glucan composition produced by a method of producing the glucan composition as described later.

The immunostimulator may have a total carbohydrate content of, for example, from 60% by mass to 70% by mass, and may be preferably from 62% by mass to 66% by mass. The carbohydrates include, for example, β-glucan, α-glucan, and mannan as described above. The β-glucan content relative to the total carbohydrate content may be, for example, from 35% by mass to 70% by mass, and may be preferably 45% by mass or more, or 50% by mass or more, and may be preferably 65% by mass or less, or 60% by mass or less, from the viewpoint of promoting effects on the production of interleukins.

The immunostimulator may contain a protein. The protein content in the immunostimulator may be, for example, from 15% by mass to 25% by mass, and may be preferably from 18% by mass to 22% by mass. The ratio of the protein content to the β-glucan content in the immunostimulator may be, for example, from 0.38 to 0.1, and may be preferably 0.45 or more, or 0.5 or more, and may be preferably 0.8 or less, or 0.6 or less, from the viewpoint of promoting effects on the production of interleukins.

The immunostimulator may contain a lipid. The lipid content in the immunostimulator may be, for example, from 10% by mass to 18% by mass, and may be preferably from 12% by mass to 16% by mass. The ratio of the lipid content to the β-glucan content in the immunostimulator may be, for example, from 0.38 to 0.4, and may be preferably 0.39 or more, and may be preferably 0.4 or less, from the viewpoint of promoting effects on the production of interleukins.

The immunostimulator may contain ash. The ash content in the immunostimulator may be, for example, from 1.8% by mass to 3% by mass, and preferably may be 1.9% by mass or more, and may be 2.2% by mass or less. The ratio of the ash content to the β-glucan content in the immunostimulator may be, for example, from 0.04 to 0.065, and may be preferably 0.05 or more, and may be preferably 0.06 or less, from the viewpoint of promoting effects on the production of interleukins.

The immunostimulator may contain water-insoluble components, and the water-insoluble components may contain an active component. The immunostimulator may substantially consist of water-insoluble components. As used herein, the term "substantially" means that water-soluble components inevitably mixed in are not excluded, and that the water-soluble components content is, for example, less than 5% by mass, or less than 1% by mass. The immunostimulator may contain water-insoluble components and water-soluble components. When the immunostimulator contains water-soluble components, the content may be, for example, from 1% by mass to less than 50% by mass, and may be preferably 40% by mass or less, or 20% by mass or less, and may be preferably 5% by mass or more. It is noted that, as used herein, the term "water-insoluble" means that the solubility in 100 g of water at 25°C is less than 1 g, and the term "water-soluble" means that the solubility in 100 g of water at 25°C is 1 g or more. The immunostimulator may contain, for example, a third treated material obtained by a method of producing a glucan composition described later, and may consist of a third treated material.

The immunostimulator may further contain a known component having an immunomodulating effect in addition to β-glucan as a main component. Examples of the component having an immunomodulating effect include Rubus suavissimus extract, fucoidan, Arabinoxylan, lactoferrin, catechin, chitosan, chitosan oligosaccharide, chitin oligosaccharide, L-ascorbic acid, and coenzyme Q10 (including reduced form).

The immunostimulator may be mixed, as necessary, with any components on which it is known that they can generally be used in pharmaceuticals, foods, feeds, and the like, such as water, fats and oils, sugars, vitamins, sweeteners, seasonings, acidifiers, preservatives, flavorings, colorings, excipients, fillers, binders, thickeners, stabilizers, emulsifiers, and pH adjusters.

The immunostimulator may be in any form, for example, a solid such as powder or granules, a liquid, or a paste. The dosage form of the immunostimulator can be selected as appropriate depending on the method of administration and the target of administration. Examples of the dosage forms include tablets, capsules, granules, troche, dispersions, solutions, and other formulations for oral administration. These formulations may be manufactured according to known methods using additives such as excipients, lubricants, binders, disintegrants, stabilizers, correctives, and diluents. The immunostimulator may also be contained as a component of food compositions such as health foods and health supplements, beverage compositions, and feeds.

When the immunostimulator is administered to a subject in need thereof, such as a vertebrate or invertebrate, it produces an immunostimulating effect in the subject. This may prevent onsets of infectious diseases in the subject. In other words, the immunostimulator may be applied to methods of preventing infectious diseases in a subject. Vertebrate subjects to which the immunostimulator is administered include mammals, birds, and fish. The mammals may include humans or may be non-human mammals. The non-human mammals include pigs, cattle, horses, sheep, and monkeys, as well as pets such as dogs and cats. The birds include poultry, laying hens, turkeys, ducks, and pigeons. The fish includes Salmonidae fish such as salmon, trout, landlocked salmon, char, and Japanese huchen; carp, crucian carp, tilapia, catfish, sea bass, yellowtail, amberjack, young yellowtail, flounder, sea bream, sea bream, tuna, and eel. Invertebrate subjects to which the immunostimulator is administered include, for example, arthropods such as crabs and shrimp; echinoderms such as sea urchins and sea cucumber; mollusks such as octopus, squid, and shellfish; and protochordate such as sea squirt.

When the immunostimulator is administered as a pharmaceutical agent or food or beverage, for example, 1 mg to 500 mg (dry weight)/kg body weight per dose may be orally administered once to several times per day. When used as a food, beverage, feed, or the like, 0.001 g to 1 g of the immunostimulator may be added per 100 g of the food, beverage, feed, or the like.

When the immunostimulator is administered as feed, for example, for mammals, birds, and fish, a mixture containing 0.001 to 1% by mass of the immunostimulator relative to the feed to be administered may be administered once to several times per day.

### Method of Producing Glucan Composition

The method of producing a glucan composition include:
a washing step for washing yeast cell walls under alkaline conditions to obtain a first treated material,
a heating step for heating the first treated material under alkaline conditions to obtain a second treated material, and
a collecting step for collecting a third treated material as a solid content from the second treated material. The resulting third treated material has a β-glucan content of from 25% by mass to 45% by mass, and has excellent immunostimulating effect. The third treated material also has excellent promoting effects on the production of interleukins, and particularly has excellent promoting effects on the production of interleukin 8.

A glucan composition having excellent immunostimulating effect may be efficiently produced by washing yeast cell walls under alkaline conditions, and then hydrolyzing the yeast cell walls under alkaline conditions. This may be considered because, for example, components contributing to the immunostimulating effect are efficiently concentrated by the washing step.

The yeast cell walls used in the method of producing a glucan composition may be a commercially available product, or may be, for example, obtained by pretreatment of yeasts as raw materials. The pretreatment step for pretreating yeasts as raw materials may include, for example, a preliminary decomposition step that is, for example, an autodigestion step for autodigestion of yeasts to obtain an autodigestion product, a hot water extraction step for hot water extraction of yeasts to obtain a hot water extraction product, or an enzyme treatment step for enzyme treatment of yeasts to obtain an enzyme treatment product alone, or a combination thereof; and a separation step for centrifugation of the preliminary decomposition product after the preliminary decomposition step to obtain yeast cell walls.

Examples of the yeasts as raw materials include Saccharomyces, Schizosaccharomyces, Kluyveromyces, Candida, Pichia, and Torulopsis yeasts, and preferably at least one selected from the group consisting of them is contained, and more preferably at least Saccharomyces yeasts are contained. The Saccharomyces yeasts include food-grade yeasts such as beer yeasts and baker's yeasts. Examples of the food-grade yeasts include brewing yeasts, such as beer yeasts, sparkling wine yeasts, miscellaneous liquor yeasts, refined sake yeasts, wine yeasts, and whisky yeasts; baker's yeasts; and torula yeasts.

In the autodigestion step, proteins and the like of the yeasts are decomposed by proteases and the like of the yeasts themselves to obtain an autodigestion product. The autodigestion step is performed, for example, with adjustment of the pH in the range from 2 to 7, and the temperature in the range from 40°C to 65°C, for a time period of from 1 hour to 48 hours. In the separation step, the autodigestion product is centrifuged to obtain a fraction containing yeast cell walls as the heavy liquid. For the centrifugation, for example, a nozzle centrifuge, an intermittent discharge centrifuge, a Sharples centrifuge, or other centrifuges can be used in industrial production. The centrifugation conditions can be adjusted as appropriate as long as insoluble components in the autodigestion product can be collected.

### Washing Step

In the washing step, yeast cell walls are washed under alkaline conditions to obtain a first treated material. The washing method may be any method as long as it can remove at least a part of proteins, amino acids, and the like remaining in the yeast cell walls. The washing method may include, for example, mixing yeast cell walls and a liquid medium to prepare a wash mixture containing yeast cell walls, and removing at least a part of the liquid medium from the wash mixture. The liquid medium may contain at least water and an alkaline substance. The method of removing the liquid medium from the wash mixture may be selected as appropriate from commonly used solid-liquid separation methods. The solid-liquid separation methods include, for example, centrifugation methods with yeast separators and the like, and methods using filters and ultrafilters.

The washing of yeast cell walls may include adjusting the pH of the wash mixture containing yeast cell walls in the range from pH 8 to pH 14, preferably from pH 9.5 to pH 12, and subjecting the resulting product to solid-liquid separation using a centrifuge (e.g., yeast separator) with addition of water. The wash mixture may contain at least water as a liquid medium. The wash mixture containing yeast cell walls may have a desired pH adjusted by adding an alkaline substance to the wash mixture. Examples of alkaline substances include alkali metal hydroxides, such as sodium hydroxide and potassium hydroxide; alkaline earth metal hydroxides, such as calcium hydroxide; and alkali metal carbonates, such as sodium carbonate. An alkaline substance may be used as an aqueous solution for pH adjustment.

The solid-liquid separation in the washing of yeast cell walls may include centrifugation with addition of water. For the addition of water, only water may be used, or an aqueous alkaline solution containing an alkaline substance may be used. The aqueous alkaline solution may have a pH, for example, in the range from pH 8 to pH 14, and preferably from pH 9.5 to pH 12.

The washing temperature in the washing step may be, for example, within a temperature range from 4°C to 95°C, and preferably from 20°C to 50°C. The washing may be performed, for example, until the liquid after solid-liquid separation by centrifugation becomes colorless to pale, or transparent.

The first treated material obtained in the washing step may be water-insoluble, and may be a water-insoluble solid. The first treated material may be in a slurry form containing yeast cell walls that are water-insoluble solids.

### Heating Step

In the heating step, the first treated material is heated under alkaline conditions to obtain a second treated material. The heating under alkaline conditions may be alkaline hydrolysis of the first treated material. The heating step may include obtaining a mixture containing the first treated material, an alkaline substance, and a liquid medium, raising the temperature of the mixture to a predetermined temperature, and keeping the mixture at the predetermined temperature for a predetermined time period. During the heating step, the mixture may be stirred, or left to stand. In keeping the mixture at a predetermined temperature, temperature variation, for example, of about ±10°C, preferably of about ±5°C, is permittable.

The alkaline conditions under which the first treated material is heated may be, for example, from pH 8 to pH 14, and may be preferably pH 9 or more, and may be preferably pH 12 or less. Heating under alkaline conditions at a relatively low pH tends to result in a glucan composition having further excellent immunostimulating effect. The pH of the first treated material, for example, when the first treated material is a slurry containing yeast cell walls, may be adjusted to a desired pH by adding an alkaline substance as described above to the slurry. The addition method may be in a batch manner or continuous manner. The amount of the alkaline substance added for adjustment to the pH described above may be, for example, from 0.01% by mass to 1% by mass, and may be preferably 0.05% by mass or more, and may be preferably 0.5% by mass or less, relative to that of the mixture.

The heating temperature may be, for example, from 60°C to 120°C, and may be preferably 70°C or more, or 75°C or more, and may be preferably 110°C or less, or 95°C or less. The heating time may be, for example, from 3 hours to 24 hours, and may be preferably 12 hours or more, and may be preferably 20 hours or less. The atmosphere under which the heating is performed may be, for example, air atmosphere.

The second treated material obtained in the heating step may be, for example, in a slurry form containing a water-insoluble solid, and the solid content may be from 10% by mass to 20% by mass.

### Collecting Step

In the collecting step, a third treated material containing a solid content is collected from the second treated material obtained in the heating step. The second treated material to be subjected to the collecting step may be neutralized or in an alkaline state. The solid content may be collected from the second treated material by solid-liquid separation of the second treated material. For the solid-liquid separation, for example, centrifugation methods, and methods using filters and ultrafilters may be used. For the centrifugation, for example, a yeast separator, a nozzle centrifuge, an intermittent discharge centrifuge, a Sharples centrifuge may be used.

The third treated material may be collected as a wet cake containing water, or may be collected as a slurry containing a solid content. The solid content in the third treated material may be, for example, 5% by mass or more, and may be preferably 10% by mass or more, or more than 20% by mass.

The method of producing a glucan composition may further include a drying step for drying the third treated material. For the drying step, for example, a spray dryer, a freeze dryer, or a drum dryer may be used. The method of producing a glucan composition may include a sterilization step for sterilizing the third treated material before the drying step. The sterilization step may be performed, for example, by heating with a UHT sterilizer at 120°C or higher for 10 seconds to 60 seconds, which may be adjusted as appropriate to meet the desired quality.

The glucan composition obtained by the method of producing a glucan composition may be an immunostimulator because of its excellent immunostimulating effect. The glucan composition may be an interleukin production promoter because of its excellent promoting effects on the production of interleukins, and may be preferably an interleukin 8 production promoter.

In addition to obtaining a glucan composition, the method of producing a glucan composition may include obtaining a yeast-derived water-soluble polysaccharide. The yeast-derived water-soluble polysaccharide may be collected from a supernatant solution separated from the second treated material. Specifically, the yeast-derived water-soluble polysaccharide can be obtained by a method including heating a supernatant solution under acid conditions, removing the coagulated product produced by the heating, and drying the solution from which the coagulated product has been removed.

The resulting yeast-derived water-soluble polysaccharide has excellent water solubility, low viscosity when it is in a state of an aqueous solution, and a high mannan content. Thus, methods of producing a glucan composition may include a method of producing a mannan composition. The mannan content in the yeast-derived water-soluble polysaccharide may be, for example, from 40% by mass to 80% by mass.

The heating of the supernatant solution under an acidic condition may be performed, for example, at a pH from 2.5 to 5, preferably under an acidic condition of pH 3.5 to 4.5. The heating temperature may be, for example, at a temperature from 90°C to 120°C, preferably from 95°C to 115°C. The heating time may be, for example, for a period of time from 15 seconds to 120 seconds, preferably from 30 seconds to 90 seconds.

In removing the coagulated product produced by the heating, for example, centrifugation, diatomaceous earth filtration, and ultrafilter may be performed. In drying the solution from which the coagulated product has been removed, for example, a spray dryer, freeze dryer, or drum dryer may be used.

### Immunostimulatory Method

The immunostimulatory method may include administering an immunostimulator containing the glucan composition described above to a subject. The subject may be, for example, a vertebrate or invertebrate. Details of the subject and dosage are as previously described.

In another aspect, the present invention includes use of a glucan composition in the manufacture of an immunostimulator used in an immunostimulatory method, use of a glucan composition in an immunostimulatory method, and a glucan composition used in an immunostimulatory method.

### EXAMPLES

The present invention will be described in more detail with reference to Examples, but is not limited thereto.

### Example 1

Yeast cell walls (solid content 15%, derived from autodigestion products from yeasts) derived from beer yeasts belonging to the Saccharomyces genus, produced in Tochigi Koganei Plant of Asahi Group Foods, Ltd. were prepared, and a yeast glucan composition was obtained according to the following methods.

Yeast cell walls were washed under alkaline conditions with addition of water and sodium hydroxide using a yeast separator to obtain a first treated material with pH adjusted in the range from 10 to 11. The mixture was heated at a temperature from 80°C to 90°C for 17 hours to obtain a second treated material. The resulting second treated material was centrifuged to obtain a third treated material as the heavy liquid. The resulting third treated material was dried with a drum dryer to obtain a glucan composition (YG35) of Example 1.

### Comparative Example 1

The yeast cell walls prepared in Example 1 were dried to obtain a glucan composition (YCW) of Comparative Example 1.

### Comparative Example 2

The glucan composition (YG35) of Example 1 was purified by proteases and then lyophilized to obtain a glucan composition (YG45) of Comparative Example 2.

### Comparative Example 3

The glucan composition (YG45) of Comparative Example 2 was purified to remove lipids by using an organic solvent, and then lyophilized to obtain a glucan composition (YG55) of Comparative Example 3.

A component analysis was performed on the glucan composition obtained above. Table 1 shows the results. In Table 1, the β-glucan, α-glucan, and mannan contents are as parts of carbohydrates relative to the total glucan composition, and the glycogen content is as a part of α-glucan relative to the total glucan composition.

**[Table 1]**

| | | YCW | YG35 | YG45 | YG55 |
|---|---|---|---|---|---|
| Protein (mass%) | | 25.9 | 19.9 | 16.4 | 18.9 |
| Lipid (mass%) | | 8.2 | 14.1 | 18.5 | 5.3 |
| Ash (mass%) | | 1.5 | 2.0 | 1.5 | 1.7 |
| Carbohydrate (mass%) | | 64.4 | 64.0 | 63.6 | 74.0 |
| | β-Glucan (mass%) | 22.0 | 35.8 | 46.1 | 54.9 |
| | α-Glucan (mass%) | 12.4 | 9.9 | 6.5 | 6.4 |
| Glycogen (mass%) | | 3.5 | 6.6 | 2.6 | 2.7 |
| | Mannan (mass%) | 24.3 | 11.1 | 3.5 | 4.0 |
| Glucan (β+α) (mass%) | | 34.40 | 45.70 | 52.60 | 61.30 |
| Manna/β-Glucan | | 1.10 | 0.31 | 0.08 | 0.07 |
| α-Glucan/β-Glucan | | 0.56 | 0.28 | 0.14 | 0.12 |
| Glycogen/β-Glucan | | 0.16 | 0.18 | 0.06 | 0.05 |

### Preparation of β-glucan Sample

The glucan compositions obtained above were ultrasonicated for 3 minutes while ice-cooling to obtain dispersions. The resulting dispersions were subjected to pepsin treatment at 39°C for 2 hours by adding pig-derived pepsin. Then, pancreatin treatment was performed at 39°C for 4 hours by addition of pig-derived pancreatin to prepare β-glucan samples.

### (1) Isolation of Neutrophils

Peripheral blood was collected from jugular veins of six 10-week-old weanling piglets to heparin tubes. Thereafter, neutrophils were separated from lymphocytes, monocytes, platelets, and the like by a discontinuous density gradient centrifugation method using 68% and 75% Percoll solutions. The erythrocytes were lysed with 74.7% ammonium chloride and centrifuged at 350×g and 18°C for 10 minutes. The precipitate was then washed with RPMI1640 medium three times and resuspended to 10⁶ cells/ml.

### (2) Measurement of Production of IL-8

The isolated neutrophils were resuspended to RPMI1640 medium and plated to 24-well multi-well plate to 2×10⁵ cells/well. Similarly, peripheral blood mononuclear cells (PBMCs) were plated to 2×10⁷ cells/well. Thereafter, the cells were incubated at 5% CO₂ and 37°C for 2 hours to allow the neutrophils or monocytes in the PBMCs to adhere, and then the supernatant was removed. The β-glucan samples were added to a concentration of 20 µg/ml and incubated for 24 hours. Then, the supernatants were collected, and the IL-8 production was measured using a commercially available ELISA kit.

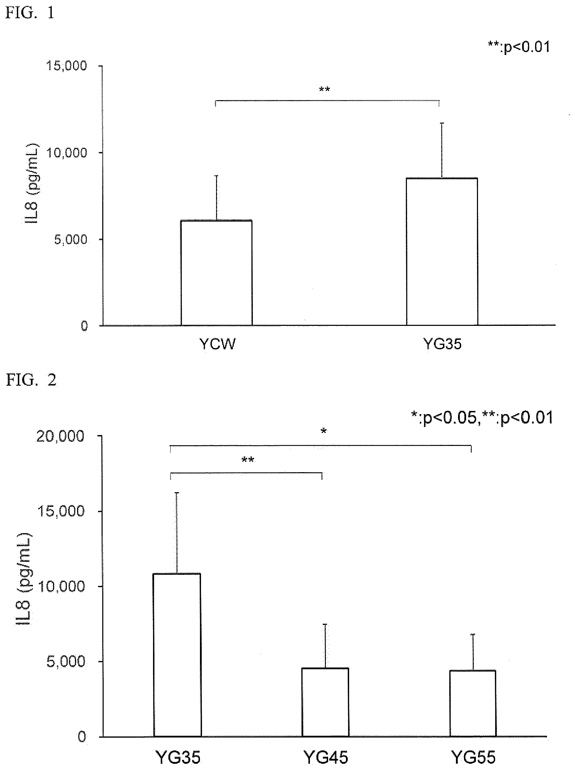

FIG. 1 shows the results obtained by using the glucan compositions of Example 1 and Comparative Example 1. A t-test showed a significant difference (p < 0.01). FIG. 2 shows the results obtained by using the glucan compositions of Example 1 and Comparative Examples 2 and 3. One-way analysis of variance analysis was performed to find significant differences (p<0.05) for all the comparisons. Thus, multiple comparison of all groups by the Bonferroni method was performed to find that Example 1 showed a significant difference (p<0.01) from Comparative Example 2 and a significant difference (p<0.05) from Comparative Example 3.

As demonstrated above, the glucan composition (YG35) containing about 35% by mass of β-glucan exhibits high immunostimulating effect. It is also demonstrated that the glucan composition (YG35) exhibits significantly higher immunostimulating effect than the glucan composition (YG45) and glucan composition (YG55) that are further purified.

The disclosure of JP 2021-086394 A filed on May 21, 2021, is incorporated herein by reference in its entirety. All documents, patent applications, and technical standards described herein are incorporated herein by reference to the same extent as if the individual documents, patent applications, and technical standards were specifically and individually stated to be incorporated by reference.

## Claims

1. An immunostimulator comprising β-glucan derived from yeast cell walls,
wherein the β-glucan content is from 25% by mass to 45% by mass.

2. The immunostimulator according to claim 1, further comprising mannan, wherein the mannan content in the immunostimulator is from 5% by mass to 20% by mass.

3. The immunostimulator according to claim 2, wherein the ratio of the mannan content to the β-glucan content is from 0.1 to less than 1.

4. The immunostimulator according to any one of claims 1 to 3, further comprising α-glucan, wherein the α-glucan content in the immunostimulator is from 7% by mass to 12% by mass.

5. The immunostimulator according to claim 4, wherein the ratio of the α-glucan content to the β-glucan content is from 0.15 to 0.55.

6. The immunostimulator according to claim 4 or 5, wherein
the α-glucan includes glycogen, and
the glycogen content in the immunostimulator is from 4% by mass to 10% by mass.

7. The immunostimulator according to claim 6, wherein the ratio of the glycogen content to the β-glucan content is 0.16 to 0.5.

8. An interleukin 8 production promoter, comprising β-glucan derived from yeast cell walls,
wherein the β-glucan content is from 25% by mass to 45% by mass.

9. A method of producing a glucan composition, comprising:
washing yeast cell walls under alkaline conditions to obtain a first treated material,
heating the first treated material under alkaline conditions to obtain a second treated material, and
collecting a third treated material as a solid content from the second treated material,
wherein in the third treated material, the β-glucan content is from 25% by mass to 45% by mass.

10. The production method according to claim 9, wherein the heating under alkaline conditions is performed at a pH from 8 to 14 and at a temperature from 70°C to 110°C.

11. The production method according to claim 9 or 10, wherein the yeast cell walls comprise cell walls derived from a brewing yeast or a baker's yeast.

12. The production method according to any one of claims 9 to 11, wherein
the washing under alkaline conditions comprises:
adjusting the pH of a wash mixture containing yeast cell walls to a pH of 8 to 14, and
separating the solid and liquid using a centrifuge with addition of water.

13. The production method according to any one of claims 9 to 12, wherein the third treated material is collected by subjecting the second treated material to solid-liquid separation by centrifugation.

14. The production method according to any one of claims 9 to 13, wherein the glucan composition is an immunostimulator.

15. The production method according to any one of claims 9 to 14, wherein the glucan composition is an interleukin 8 production promoter.
